# EUROPEAN PATENT APPLICATION

(11) **EP 2 311 793 A1**
(43) Date of publication of application: **20.04.2011**
(21) Application number: 10184848.9
(22) Date of filing: 19.12.2005
(51) Int. Cl.: C07C 215/60, C07C 213/10, A61K 9/12, A61K 31/137, A61P 11/06

(54) **Crystalline levosalbutamol sulphate (Form II)**

(30) Priority: 17.12.2004 IN MU13562004; 14.01.2005 IN MU00402005; 24.03.2005 IN MU03432005
(62) Divisional of application: 05843722.9
(71) Applicant: Cipla Ltd., Mumbai 400 008 (IN)
(72) Inventor: Lulla, Amar, Mumbai 400 005 Maharashtra (IN); Malhotra, Geena, Mumbai, 400 010 Maharashtra (IN); Rao, Dharmaraj, Ramchandra, 400 601 Maharashtra (IN); Kankan, Rajendra, Narayanrao, Mumbai, 400 084 Maharashtra (IN); Chaudhary, Alka, 410 206 Maharashtra (IN)
(74) Representative: Turner, Craig Robert

(57) **Abstract**

The invention provides crystalline levosalbutamol sulphate polymorphic Form II. Form II is characterised by a powder XRD pattern with peaks at 8.7, 9.6, 15.2, 15.7, 19.1, 27.2, 30.7 ± 0.2 degrees 2 theta. Processes for making polymorphic Form II and pharmaceutical composition comprising it are provided. A preferred formulation comprises a pharmaceutical composition comprising crystalline levosalbutamol sulphate form II, a glucocorticoid and a pharmaceutically acceptable carrier.

## Description

The present invention relates to crystalline levosalbutamol sulphate, polymorphs thereof, processes for making the crystalline material, and compositions thereof.

It also relates to a pharmaceutical composition comprising a therapeutically effective isomer of salbutamol in combination with a glucocorticoid, the composition being useful for the treatment of respiratory disorders including bronchoconstriction, asthma, COPD and related disorders thereof.

Asthma is described as a chronic disease that involves inflammation of the pulmonary airways and bronchial hyperresponsiveness that results in the clinical expression of a lower airway obstruction that usually is reversible. The pathophysiology of asthma or related disorders involves bronchoconstriction resulting from bronchial smooth muscle spasm and airway inflammation with mucosal edema. Treatment of asthma and other related disorders have been known to employ β―2 agonists, also known as β-2 adrenoreceptor agonists. Such β—2 adrenoreceptor agonists are known to provide a bronchodilator effect to patients, resulting in relief from the symptoms of breathlessness. More particularly, β―2 adrenoreceptor agonists have been shown to increase the conductance of potassium channels in airway muscle cells, leading to membrane hyperpolarization and relaxation. Short-acting beta2 adrenoreceptors like salbutamol and terbutaline are recommended for the relief of acute symptoms, while long-acting agents like salmeterol, formoterol and bambuterol are used preferably in combination with other drugs for long-term asthma control.

Chronic Obstructive Pulmonary Disease (COPD) is a preventable and treatable disease state characterized by airflow limitation that is not fully reversible. COPD (Chronic Obstructive Pulmonary Disease) is an umbrella term used to describe lung disease associated with airflow obstruction. The airflow limitation is usually progressive and associated with an abnormal inflammatory response of the lungs to noxious particles or gases, primarily caused by cigarette smoking.

Bronchodilators are the mainstay of therapy for patients with established chronic obstructive pulmonary disease (COPD) but, at present, the majority of patients use β-agonists.

Salbutamol pressurized inhalation is official in the British pharmacopoeia and are used for the treatment of asthma.

Dey pharmaceutical's patent US 6,702,997 relates to an albuterol inhalation solution, system, kit and method for relieving bronchospasm in children suffering from asthma which comprises about 0.63 mg or about 1.25 mg albuterol.

US6,251,368 relates to a pharmaceutical aerosol formulation that comprises particulate medicament selected from the group consisting of salmeterol, salbutamol, fluticasone propionate, beclomethasone dipropionate and physiologically acceptable salts and solvates thereof and a fluorocarbon or hydrogen-containing chlorofluorocarbon propellant, which formulation is substantially free of surfactant is disclosed

US 5,547,994 by Sepracor describes a method for treating asthma, using the optically pure R (-) isomer of albuterol, which is substantially free of the S(+) isomer, is a potent bronchodilator for relieving the symptoms associated with asthma in individuals.

CN1413976 by Suzhou Junning New Drug Dev CT (CN), which describes the synthesis of levosalbutamol.

US patent application number US2004054215 by CIPLA Limited discloses a method for obtaining an optically pure R-isomer of albuterol.

Several methods for preparation of levalbuterol have been described in the prior art such as US patent application number 20040115136 by King Code which describes a method of preparation of levalbuterol tartrate. It further relates to levalbuterol L-tartrate possessing properties desirable for use in a metered dose inhaler.

Salbutamol (albuterol) is an antihistaminic compound and is a beta 2-adrenoceptor agonist used as a bronchodilator for the treatment of asthma and as a uterine relaxant for the suspension of premature labour. Salbutamol has been marketed as a racemic mixture, although the beta 2-agonist activity resides almost exclusively in the (R)-enantiomer. The enantioselective disposition of salbutamol and the possibility that (S)-salbutamol has adverse effects have led to the development of an enantiomerically pure (R)-salbutamol formulation known as levosalbutamol (levalbuterol) (Formula I).

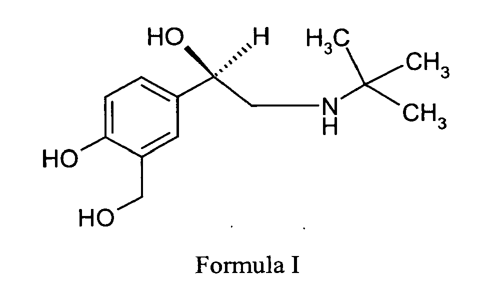

A process for the preparation of optically pure salbutamol from mono protected salbutamol precursor is disclosed in US5545745.

US2004114136 and WO2004052835 describe a process for preparing levalbuterol L-tartrate in crystalline form; a pharmaceutical composition comprising levalbuterol L-tartrate, in crystalline form; a metered dose inhaler comprising a canister containing an aerosol formulation of levalbuterol L-tartrate in crystalline form; and a method of affecting bronchodilation in a patient using levalbuterol L-tartrate, including levalbuterol L-tartrate specifically in crystalline form.

Levosalbutamol is prepared by hydrogenating R-benzyl salbutamol in the presence of palladium on carbon.

R-benzyl salbutamol can be prepared by the process described in United States patent number 5,545,745.

Studies have proved that racemic albuterol, a commonly used bronchodilator, is an exact 50:50 mixture of two enantiomers, R- and S- isomers of salbutamol. Only the R-enantiomer (levosalbutamol) is a potent β₂ —adrenoceptor stimulant, whereas the S-enantiomer (dextrosalbutamol) shows little or no adrenoceptor activity.

Among the different classes of drugs which are usually administered by inhalation for the treatment of respiratory diseases, glucocorticosteroids such as beclomethasone dipropionate (BDP), dexamethasone, flunisolide, budesonide, fluticasone propionate are of great importance. They can be administered in the form of a finely divided, i.e. micronised, powder, formulated as suspension in an aqueous phase containing any necessary surfactants and/or cosolvents; when intended to be administered in the form of metered doses of aerosol spray, they should also contain a low-boiling propellant.

The effectiveness of the administration form depends on the deposition of an adequate amount of particles at the action site. One of most critical parameters determining the proportion of inhalable drug which will reach the lower respiratory tract of a patient is the size of the particles emerging from the device. In order to ensure an effective penetration into the bronchioli and alveoli and hence ensure a high respirable fraction, the mean aerodynamic diameter (MMAD) of the particles should be lower than 5-6 microns. For nasal administration, particles with higher MMAD are required.

Fluticasone propionate is itself known from GB2088877 to have anti-inflammatory activity and to be useful for the treatment of allergic and inflammatory conditions of the nose, throat, or lungs such as asthma and rhinitis, including hay fever. Fluticasone propionate in aerosol form, has been accepted by the medical community as useful in the treatment of asthma and is marketed under the trademarks Flovent I and "Flonase". Fluticasone propionate may also be used in the form of a physiologically acceptable solvate.

HK1009406 relates to a metered dose inhaler for dispensing an inhalation drug formulation comprising fluticasone propionate, or a physiologically acceptable solvate thereof, and a fluorocarbon propellant, optionally in combination with one or more other pharmacologically active agents or one or more excipients.

We have appreciated that the use of a combination of salbutamol or a physiologically acceptable salt thereof and inhaled corticosteroid has clinical advantages in the treatment of COPD over the use of salbutamol alone or corticosteroid alone.

U.S Pat. No. 6013245 relates to a pharmaceutical aerosol formulation which comprises particulate anhydrous beclomethasone dipropionate together with 1,1,1,2,3,3,3-heptafluoro-n-propane as propellant, which formulation is free of surfactant. The formulation may also contain salbutamol and includes a canister suitable for delivery and a method of treating respiratory disorders administering the formulation by inhalation.

U.S. Pat. No. 2004136920 relates to aerosol formulations to be administered as inhalation and which comprises particulate salbutamol and physiologically acceptable salts and solvates thereof and a fluorocarbon or hydrogen-containing chlorofluorocarbon propellant, substantially free of surfactant. The patent also describes a method of treating respiratory disorders which comprises administration by inhalation of an effective amount of a pharmaceutical aerosol formulation as defined is also described.

The present invention aims to provide a potent pharmaceutical composition comprising a therapeutically effective isomer of salbutamol or a salt, solvate, ester, derivative or polymorph thereof in combination with an inhaled corticosteroid.

The object of the present invention is to provide a pharmaceutical composition comprising at least two drugs, one of which is therapeutically effective isomer of salbutamol or a salt, solvate, ester, derivative or polymorph thereof in combination with an inhaled corticosteroid and a pharmaceutical acceptable carrier or excipient and optionally one or more other therapeutic agents.

A further object of the present invention is to provide a pharmaceutical composition comprising a therapeutically effective isomer of salbutamol or a salt, solvate, ester, derivative or polymorph thereof that avoids side effects associated with higher racemic dosages.

A still further object to provide a method for the manufacture of the pharmaceutical composition comprising the therapeutically effective isomer of salbutamol and in combination with an inhaled corticosteroid.

Yet another object of the present invention is to provide a method for the treatment in a mammal, such as a human, of respiratory disorders such as asthma, disorders resulting in bronchoconstriction, which method comprises administration of a therapeutically effective amount of a pharmaceutical composition according to present invention.

An object of the present invention is to provide a method for decreasing side effects of a drug combination comprising at least two drugs in a patient, comprising the step of: administering by inhalation to a patient in need thereof an effective amount of a pharmaceutical composition comprising at least two drugs, and a propellant.

According to the present invention there is provided a pharmaceutical composition comprising a therapeutically effective isomer of salbutamol or a salt, solvate, ester, derivative or polymorph thereof, a glucocorticoid and a pharmaceutically acceptable carrier or excipient and optionally one or more other therapeutic agents.

There is also provided a process for the manufacture of a pharmaceutical composition comprising a therapeutically effective isomer of salbutamol or a salt, solvate, ester, derivative or polymorph thereof in combination with a glucorticoid drug along with at least one pharmaceutically acceptable carrier, which method comprises mixing the ingredients to form the said composition.

The invention also provides a composition of the invention for use as a medicament.

The composition of the invention is also provided for treating respiratory disorders and related conditions, including bronchoconstriction, asthma and COPD.

The present invention also provides for a method for the treatment in a mammal, such as a human, of respiratory disorders such as asthma, disorders resulting in bronchoconstriction, and chronic obstructive pulmonary disease (COPD) which method comprises administration of a therapeutically effective amount of a pharmaceutical composition according to present invention.

Beta2 adrenoreceptors are known to provide a bronchodilator effect to patients by acting on the β-2 adrenergic receptors in the airway smooth muscles and the bronchial smooth muscles, resulting in relief from the symptoms of breathlessness. More particularly, they have been shown to increase the conductance of potassium channels in airway muscle cells, leading to membrane hyperpolarization and relaxation. They are thus preferred in case of asthma treatment that requires the dilation of the bronchial smooth muscles and relieves the patient of breathlessness associated with asthma. More particularly the short acting beta2 adrenoreceptors are very useful since they provide a quicker onset of action and hence faster relief.

The present invention relates to one such short acting beta2 adrenoreceptor, salbutamol. The salbutamol is available as a racemic mixture comprising R and S form. But only the R-enantiomer (levosalbutamol) is a potent β₂ ―adrenoceptor stimulant, whereas the S-enantiomer (dextrosalbutamol) shows little or no adrenoceptor activity. The bronchodilatory property of racemic salbutamol is attributable entirely to (R)- salbutamol, which has an approximately 100 fold greater binding affinity for beta₂ receptors as compared to (S)- salbutamol. In vitro, (S)-salbutamol has been reported to promote intracellular Ca ²⁺ influx in airway smooth muscle-cells and augment cholinergic activation of airway smooth muscles. Thus in the absence of (R)-salbutamol, (S)-salbutamol has the potential to induce bronchoconstriction in asthmatic patients. This divergent pharmacology accentuates the need of levosalbutamol over racemic salbutamol in the treatment of asthma and other airway diseases.

Also levosalbutamol is a more potent bronchodilator when administered as the single enantiomer compared with the same amount in a racemic mixture. Levosalbutamol produces comparable efficacy at nearly one-fourth the dose of racemic salbutamol, simultaneously reducing the beta-mediated side effects.

Accordingly the present invention further provides aerosol formulations in accordance with the invention which contain two or more particulate medicaments. Medicaments may be selected from suitable combinations of the medicaments mentioned hereinbefore or may be selected from any other suitable drug useful in inhalation therapy. Preferably, the medicament may be presented in a form which is substantially completely insoluble in the selected propellant.

Appropriate medicaments may thus be selected from, for example, analgesics, e.g. codeine, dihydromorphine, ergotamine, fentanyl or morphine; anginal preparations, e.g. diltiazem; antiallergics, e.g. cromoglycate, ketotifen or nedocromil; antiinfectives e.g. cephalosporins, penicillins, streptomycin, sulphonamides, tetracyclines and pentamidine; antihistamines, e.g. methapyrilene; anti-inflammatories, e.g. flunisolide, budesonide, tipredane or triamcinolone acetonide; antitussives, e.g. noscapine; bronchodilators, e.g. ephedrine, adrenaline, fenoterol, formoterol, isoprenaline, metaproterenol phenylephrine, phenylpropanolamine, pirbuterol reproterol rimiterol, terbutaline, isoetharine, tulobuterol orciprenaline, or (-)-amino-3,5-dichloro-[alpha]-[[[6-[2-(2-pyridinyl)ethoxy]hexyl] amino]methyl]benzenemethanol; diuretics, e.g. amiloride; anticholinergics e.g. ipratropium, atropine or oxitropium; hormones, e.g. cortisone, hydrocortisone or prednisolone; xanthines e.g. aminophylline, choline theophyllinate, lysine theophyllinate or theophylline; and therapeutic proteins and peptides, e.g. insulin or glucagon. It will be clear to a person skilled in the art that, where appropriate, the medicaments may be used in the form of salts (e.g. as alkali metal or amine salts or as acid addition salts) or as esters (e.g. lower alkyl esters) or as solvates (e.g. hydrates) to optimise the activity and/or stability of the medicament and/or to minimise the solubility of the medicament in the propellant.

Commercially available pharmacopoeial composition of salbutamol containing the racemic form comprises 100 to 200 mcg of salbutamol but a composition according to the present invention contains almost half the dose, or even less, and is therapeutically more effective by the use of the R form of salbutamol; levosalbutamol. Due to the reduced dosage, there are less cardiovascular complications, which are associated with higher doses of bronchodilators. Therefore the use of such therapeutically effective isomer results in increased patient compliance.

Hence the present invention provides a pharmaceutical composition comprising a therapeutically effective isomer of salbutamol or a salt, solvate, ester, derivative or polymorph thereof that avoids side effects associated with higher racemic dosages.

The term 'levosalbutamol' is used in the entire specification and claims in a broad sense to include not only levosalbutamol per se but also its pharmaceutically available salts, derivatives or polymorphs thereof. The pharmaceutically available salts of levosalbutamol include levosalbutamol sulphate, levosalbutamol tartrate, levosalbutamol hydrochloride. The salt of levosalbutamol used is preferably levosalbutamol sulphate.

The active compounds and the various derivatives thereof may be made according to procedures known in the art, as will be clear to the skilled person.

The invention employs the most active, therapeutically speaking, isomer of salbutamol. The compositions are substantially free of the less therapeutically effective isomer, meaning that this isomer will not be present in any significant amount. Suitably, such isomers will be present at no more than 10% w/w of active, more preferably 1% w/w or less. Thus, for example, compositions containing levosalbutamol, are substantially free of the S-isomer of this compound.

Whilst any suitable form of composition may be used, particularly preferred compositions are aerosol, DPI or inhalation solution/suspension formulations containing levosalbutamol (e.g. as the free base or the sulphate salt) in combination with an antiinflammatory steroid such as a beclomethasone ester (e.g. the diproprionate) or a fluticasone ester (e.g. the propionate) or an antiallergic such as cromoglycate (e.g. the sodium salt). Combinations of salbutamol and fluticasone propionate or beclomethasone dipropionate or budesonide are preferred. It will be understood that for inhalable compositions such as aerosol formulations, the actives will be provided in suitably inhalable form.

In the compositions of the invention, we prefer to use polymorphic forms of levosalbutamol sulphate named herewith as Form I, Form II and Form III. These are novel compounds and constitute a further aspect of the invention.

Accordingly, in one aspect, the invention provides crystalline levosalbutamol sulphate (Form I) is characterised by a powder XRD pattern with peaks at 10.8, 11.9, 13.0, 18.3, 28.5 ± 0.2 degrees 2 theta.

In another aspect, there is provided crystalline levosalbutamol sulphate (Form II) is characterised by a powder XRD pattern with peaks at 8.7, 9.6, 15.2, 15.7, 19.1, 27.2, 30.7 ± 0.2 degrees 2 theta.

In another aspect, there is provided crystalline levosalbutamol sulphate (Form III) is characterised by a powder XRD pattern with peaks at 5.5, 6.9, 7.3, 18.7 ± 0.2 degrees 2 theta.

The invention also provides various processes for making Form I, II and III.

A process for preparing crystalline levosalbutamol sulphate Form I, comprises a) preparing levosalbutamol in an organic solvent b) adjusting the pH by addition of sulphuric acid at from 1 to 10°C c) isolating the product (Form I) at from 0 to 10°C.

A process for preparing crystalline levosalbutamol sulphate Form I, comprises a) dissolving any form of levosalbutamol sulphate in water b) combining the solution from step a) with a water miscible organic solvent so as to cause precipitation c) isolating Form I thereon.

A process for preparing crystalline levosalbutamol sulphate Form II, comprises a) dissolving any form of levosalbutamol sulphate in water b) distilling to residue c) stripping the residue with an organic solvent d) slurrying the solid in an organic solvent e) isolating crystalline Form II.

A further process for preparing Form II comprises jet milling any other form of levosalbutamol sulphate , for example jet milling crystalline Form I.

A process for preparing crystalline levosalbutamol sulphate Form III, comprises a) preparing levosalbutamol in an organic solvent b) adjusting the pH by addition of sulphuric acid at 25 to 30° C c) isolating the product (Form III) at 25 to 30° C .

Another process for preparing Form III comprises a) dissolving any form of levosalbutamol sulphate in water b) combining the solution from step a) with a water-miscible organic solvent so as to cause precipitation c) isolating Form III therefrom at 25 to 30° C.

The invention also provides a pharmaceutical composition comprising a compound of the invention and a pharmaceutically acceptable carrier.

The novel compounds, and compositions thereof, are also provided for use as medicaments, particularly in the treatment of respiratory disorders and related conditions.

Figure 1 shows the X-ray powder diffraction pattern of levosalbutamol sulphate Form I.

Figure 2 shows an IR spectrum of levosalbutamol sulphate Form I.

Figure 3 shows the X-ray powder diffraction pattern of levosalbutamol sulphate Form II.

Figure 4 shows an IR spectrum of levosalbutamol sulphate Form II.

Figure 5 shows the X-ray powder diffraction pattern of levosalbutamol sulphate Form III.

Figure 6 shows an IR spectrum of levosalbutamol sulphate Form III.

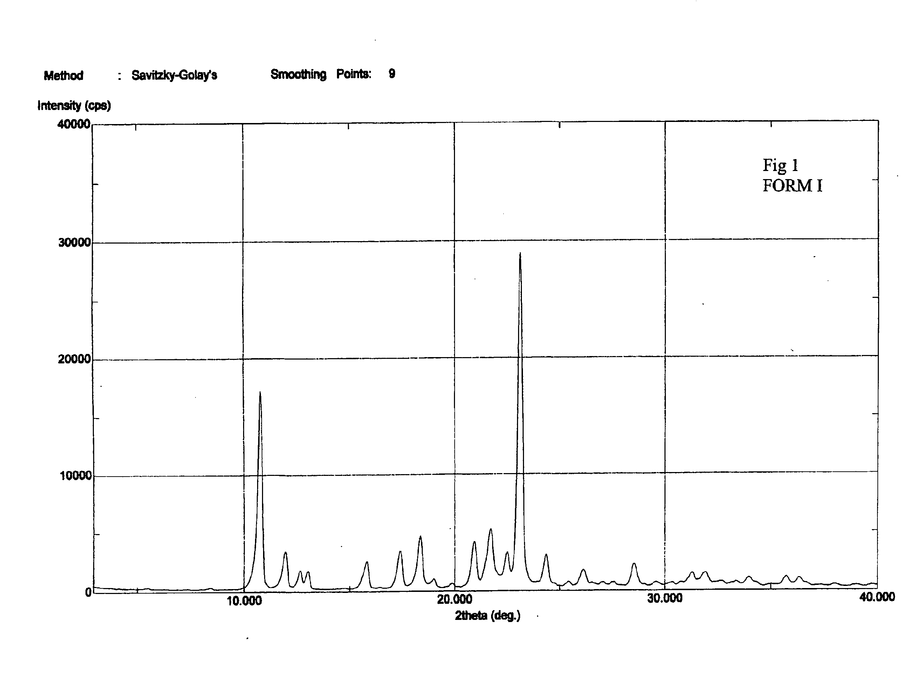

Table 1 gives the numerical XRD data for Figure 1 (Form I).

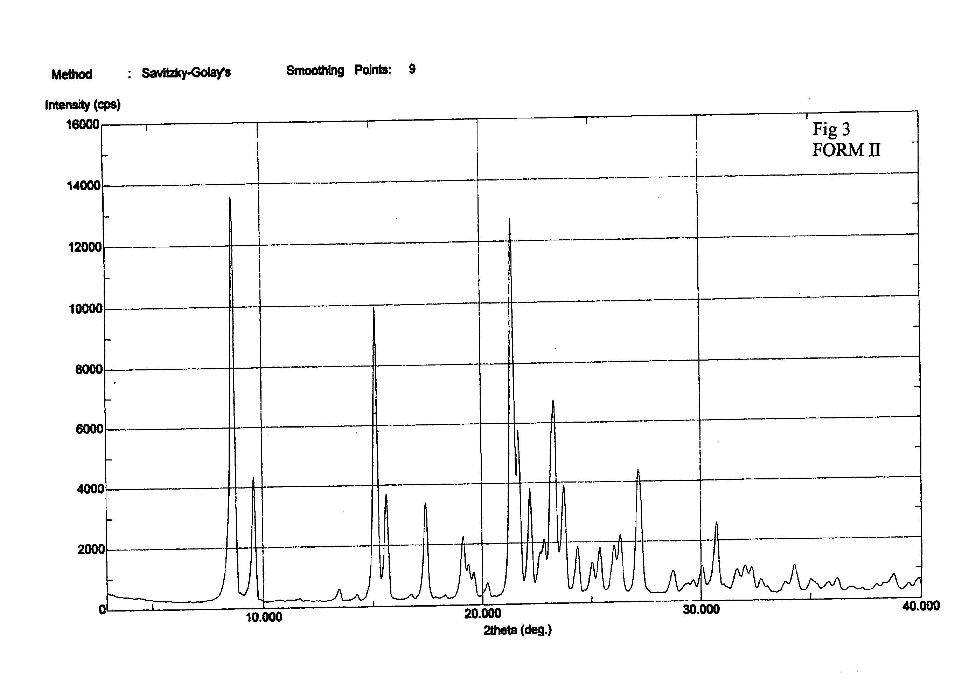

Table 2 gives the numerical XRD data for Figure 3 (Form II).

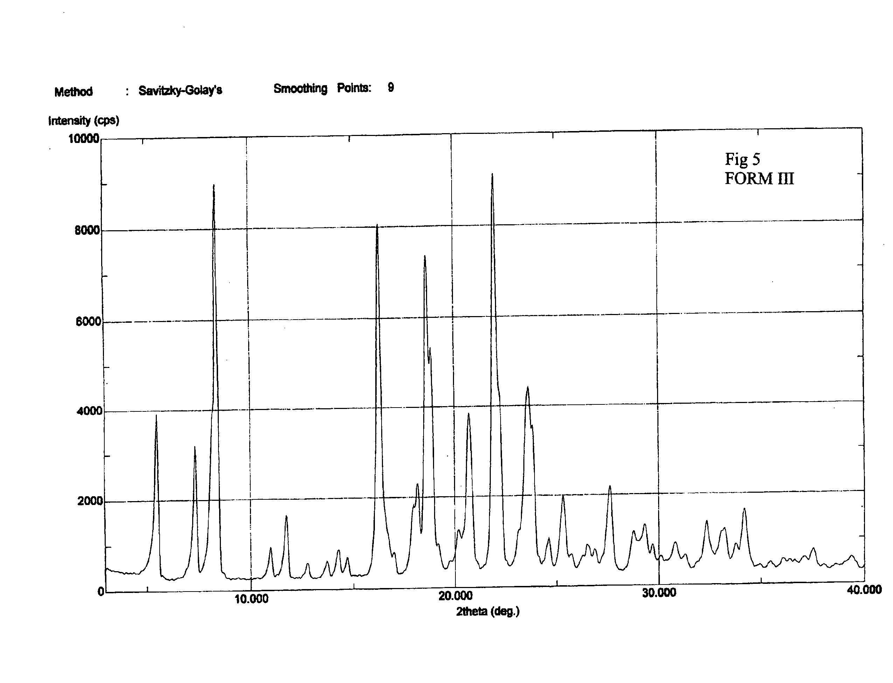

Table 3 gives the numerical XRD data for Figure 5 (Form III).

Levosalbutamol sulphate crystalline Form I is characterized by an X-ray powder diffraction pattern having significant reflections expressed as 2 theta values at about 10.781, 11.941, 13.002, 18.341, 28.541 ±0.2 degrees, as will be clear from Table 1.

The X-ray powder diffractogram of levosalbutamol sulphate crystalline Form I is shown in FIG. 1. The major peaks and their intensities of X-ray powder diffractogram are shown in Table 1. The intensities of the reflections are also expressed as percent of most intense reflection.

Other preferred significant reflections for Form I expressed as 2 theta values include 12.66, 15.819, 17.4, 20.939, 21.72, 22.5, 23.14, 24.341, 26.12, 31.28, 31.93 ± 0.2 degrees. The X-ray powder diffractograms for all the polymorphic Forms disclosed herein were collected on Rigaku d-max 2200 model X-ray diffractometer using Cu K α radiation ( λ= 1.5405 A°).

Levosalbutamol sulphate crystalline Form I is also characterised by an IR spectrum with peaks at 3568, 3307, 2980, 2799, 2561, 2458, 1615, 1508, 1440, 1380, 1342, 1258, 1200, 1112, 1082, 1029, 976, 915, 836, 793, 775, 752, 648, 617, 535, 497, 453 cm⁻¹.

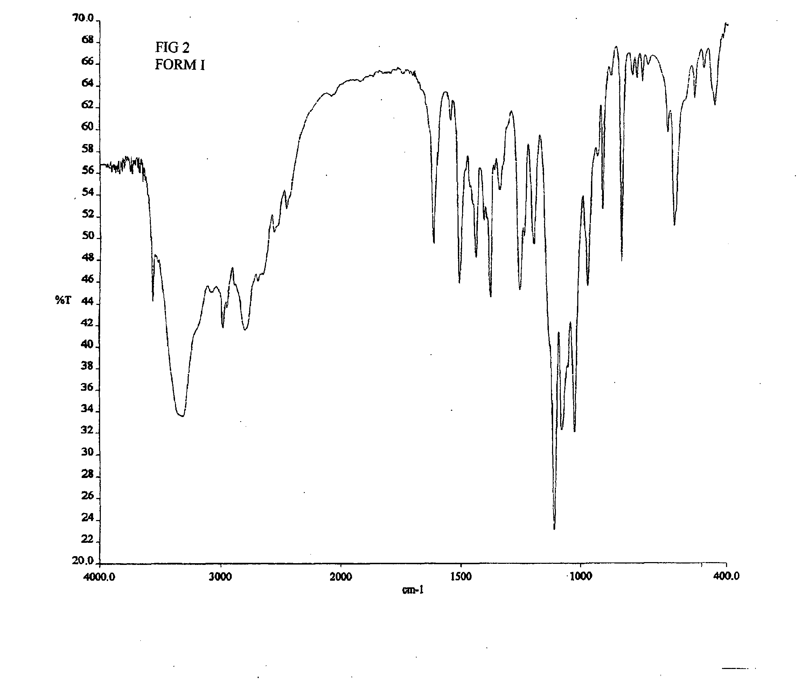

Figure 2 shows the IR spectrum for Form I. The IR spectra for all the polymorphic Forms disclosed herein were collected using the Spectrum-1 make of Perkin Elmer Sample and analysed as KBr pellets in the region of 4000-400 cm⁻¹.

In the preparation of levosalbutamol sulphate crystalline Form I, preferably R-benzyl salbutamol is hydrogenated using a catalyst, preferably a palladium on carbon catalyst, in a large volume of a suitable organic solvent. Preferably an alcoholic solvent is used, more preferably ethyl alcohol. Suitably the process is performed under hydrogen pressure, preferably at 30psi. The catalyst is preferably then filtered and the pH of the filtrate is adjusted, preferably to 5-5.5 and preferably at 0-10 °C with sulfuric acid, suitably concentrated sulphuric acid, to provide crystals, which are filtered and dried to afford levosalbutamol sulphate Form I. The product (Form I) may be obtained by isolating at 0-10° C.

Levosalbutamol sulphate crystalline Form II is characterized by an X-ray powder diffraction pattern having significant reflections expressed as 2 theta values at about 8.701, 9.636, 15.180, 15.657, 19.139, 27.199, 30.702 ± 0.2 degrees, as will be clear from Table 2.

The X-ray powder diffractogram of levosalbutamol sulphate Form II is shown in FIG. 3. The major peaks and their intensities of X-ray powder diffractogram are shown in Table 2. The intensities of the peaks are expressed as percent of most intense reflection.

Other preferred significant reflections for Form II expressed as 2 theta values include peaks at about 8.701,9.636, 15.180, 18.657, 17.44, 19.139, 21.699, 22.201, 22.837, 23.339, 23.76, 24.361, 25.022, 25.399, 26.059, 26.321, 27.199, 30.702 ± 0.2 degrees.

Levosalbutamol sulphate crystalline Form II is also characterised by an IR spectrum with peaks at 3393, 3026, 2982, 2822, 2463, 1630, 1614, 1513, 1484, 1448, 1380, 1321, 1279, 1258, 1235, 1204, 1155, 1093, 1066, 1036, 1023, 919, 900, 838, 829, 818, 808, 788, 618, 596, 540, 493, 453, 440 cm⁻¹.

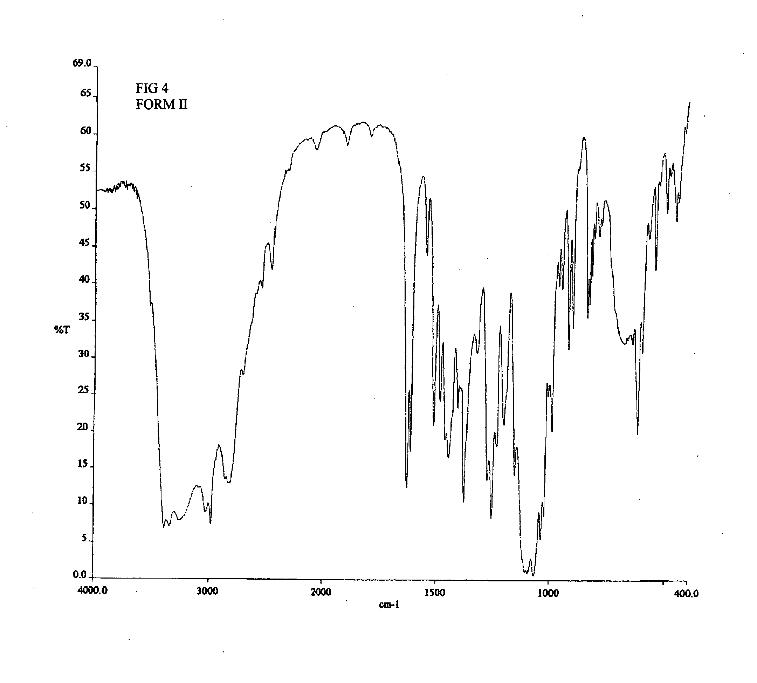

Figure 4 shows the IR spectrum for Form II.

A process for the preparation of levosalbutamol sulphate crystalline Form II, comprises dissolving any form of levosalbutamol sulphate in water and distilling it to residue. The residue is further stripped with an organic solvent, which is preferably water miscible and is preferably acetone, and the solid further slurried in a solvent, preferably the same solvent, and isolating the solid, preferably by filtering the solid and drying under vacuum to give levosalbutamol sulphate Form II.

Levosalbutamol sulphate crystalline Form III is characterized by an X-ray powder diffraction pattern having significant reflections expressed as 2 theta values at about 5.496, 6.901, 7.340, 18.660 ± 0.2 degrees, as will be clear from Table 3.

The X-ray powder diffractogram of levosalbutamol sulphate Form III is shown in FIG. 5. The major peaks and their intensities of X-ray powder diffractogram are shown in Table 3. The intensities of the peaks are also expressed as a percent of the most intense reflection.

Other preferred significant reflections for Form III expressed as 2 theta values include peaks at about 5.496, 6.901, 7.340, 8.18, 8.399, 10.978, 11.758, 14.298, 16.321, 17.98, 18.18, 18.660, 18.86, 19.189, 20.179, 20.72, 20.019, 22.219, 23.121, 23.64, 23.858, 24.638, 25.339, 27.62, 28.79, 29.319, 30.80, 32.341, 33.218, 33.781, 34.181 ± 0.2 degrees.

Levosalbutamol sulphate crystalline Form III is also characterised by an IR spectrum with peaks at 3533, 3412, 3086, 2979, 2823, 2799, 1613, 1547, 1505, 1437, 1397, 1380, 1365, 1353, 1303, 1256, 1243, 1198, 1110, 1133, 1086, 1075, 1055, 1029, 990, 949, 919, 838, 792, 737, 723, 640, 618, 563, 536, 480, 442, 425 cm⁻¹.

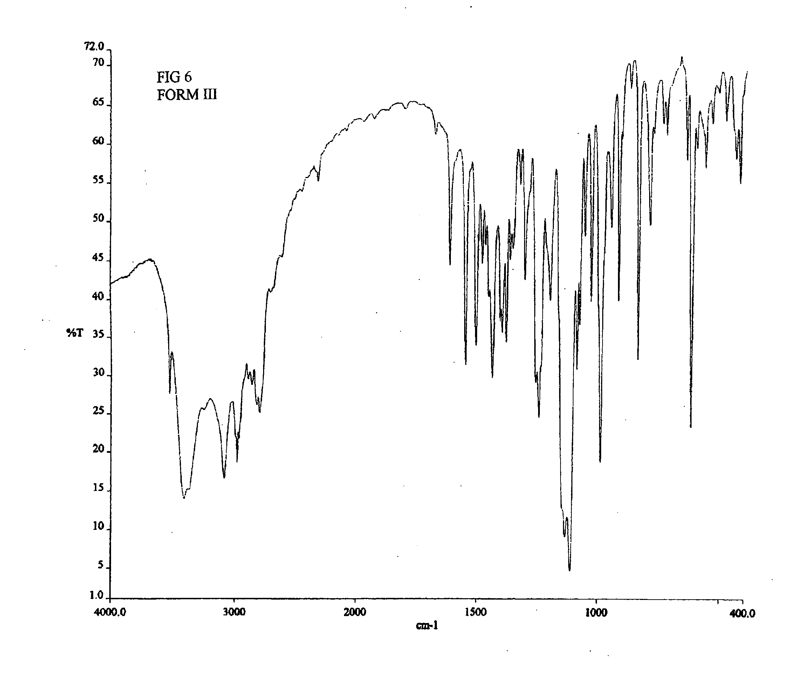

Figure 6 shows the IR spectrum for Form III.

In a process for the preparation of levosalbutamol sulphate crystalline Form III, preferably R-benzyl salbutamol is hydrogenated using a catalyst, preferably a palladium on carbon catalyst in a suitable organic solvent, preferably an alcoholic solvent, more preferably ethyl alcohol. Preferably this is done under hydrogen pressure, preferably at about 30psi. Form III can be isolated by adjusting the pH by addition of sulphuric acid at ambient temperature (25 to 30° C) and isolating the product at ambient temperature (25 to 30° C). Preferably, these steps are done by filtering the catalyst and washing, for example with denatured alcohol. The pH of the filtrate is preferably adjusted to 5-5.5 at ambient temperature (25 to 30° C) with sulfuric acid, preferably in concentrated form, to give crystals, which are filtered and dried to afford levosalbutamol sulphate Form III. The product (Form III) may be obtained by isolating at 25 to 30° C.

Another process for the preparation of levosalbutamol sulphate crystalline Form II comprises jet milling levosalbutamol sulphate. For example, crystalline levosalbutamol Form I may be jet milled so as to give Form II.

It will be understood that crystalline levosalbutamol sulphate and the polymorphic Forms thereof disclosed herein may be formulated with conventional excipients, auxiliaries and carriers into a wide variety of pharmaceutical compositions, including but not limited to tablets, capsules, pellets, caplets, MDI, DPI, and Respule formulations, and oral liquids such as syrups. Where appropriate plain or sustained release formulations may be provided. Those skilled in the art of pharmaceutical formulation will be aware of the conventional ingredients which may be employed to formulate the above compositions. Such formulations may be made in accordance with conventional manufacturing procedures.

In particular, the compounds of the present invention may be combined with one or more other pharmaceutically active compounds, as will be clear to those skilled in the art. Any suitable combination of active materials is envisaged, provided the combination is acceptable from a pharmaceutical and regulatory standpoint. The compounds of the invention may, for example be combined with corticosteroids such as fluticasone, beclomethasone or budesonide; anticholinergic agents such as ipratropium, tiotropium or atropine; mucolytic agents such as ambroxol; xanthine derivatives such as theophylline; antihistamines; analgesics, and bronchodilators. As will be clear, the additional active or actives may be provided in any suitable form, including the pharmaceutically acceptable derivatives thereof, including salts, esters, polymorphs, and the optically active forms as well as the racemates.

The invention thus provides a pharmaceutical composition comprising crystalline levosalbutamol sulphate, particularly Form I or Form II or Form III thereof, in combination with one or more pharmaceutically active compounds and, optionally, a pharmaceutically acceptable carrier.

The compositions of the present invention are preferably administered by the inhalation route so as to provide an effective amount of local action and thus avoid undesirable systemic effects. The present compositions may further comprise pharmaceutically acceptable excipients in order to provide a suitable formulation and may be made available in the form of a metered dose inhaler.

An aerosol formulation according to the present invention may optionally comprise in addition to levosalbutamol in combination with anti-inflammatory steroid or inhaled glucocorticoid and at least one propellant, other pharmaceutically acceptable agents like cosolvents, antioxidants or surfactants.

For aerosol formulations, a propellant is included in the composition. Suitable propellants include propellant 11 (dichlorodifluoromethane), propellant 12 (monofluorotrichloromethane), Propellant 114, 1,1,1,2-tetrafluoroethane (HFA134a) and 1,1,1,2,3,3,3-heptafluoropropane (HFA227), or mixtures of two or more such halogen-substituted hydrocarbons.

The aerosol formulations of the invention may be prepared by dispersal of the medicament in the selected propellant in an appropriate container, e.g. with the aid of sonication. The process is desirably carried out under anhydrous conditions to obviate any adverse effects of moisture on suspension stability.

The formulations according to the invention form weakly flocculated suspensions on standing but, surprisingly, these suspensions have been found to be easily redispersed by mild agitation to provide suspensions with excellent delivery characteristics suitable for use in pressurised inhalers, even after prolonged storage. Minimising and preferably avoiding the use of formulation excipients e.g. surfactants, cosolvents etc in the aerosol formulations according to the invention is also advantageous since the formulations may be substantially taste and odour free, and less irritant and less toxic than conventional formulations.

In a preferred embodiment of the present invention an aerosol composition may comprise a therapeutically effective isomer of salbutamol a salt, solvate, ester, derivative or polymorph thereof with an anti-inflammatory steroid or inhaled glucocorticoid and either propellant 11 or propellant 114 or a combination thereof and propellant 12.

In another preferred embodiment of the present invention the aerosol may comprise a therapeutically effective isomer of salbutamol or a salt, solvate, ester, derivative or polymorph thereof with inhaled glucocorticoid and either propellant 11 or propellant 114 or a combination thereof and propellant 12 with a surfactant.

During the trial for this formulation it was observed that in the absence of a surfactant the drug failed to form a homogenous dispersion. Various surfactants known in the art were tried like oils such as corn oil, olive oil, cottonseed oil and sunflower seed oil, mineral oils like liquid paraffin, oleic acid and also phospholipids such as lecithin, or sorbitan fatty acid esters like sorbitan oleate. Lecithin gave a comparatively good suspension quality when levosalbutamol sulphate was used in combination with fluticasone and budesonide. The preferred surfactant was oleic acid in case of levosalbutamol sulphate used in combination with beclomethasone.

The surfactant can be used in a concentration of 0.001-100% by weight of the total active material. Preferably in a range of 1%-50%. More preferably in a concentration of 5%-30%. The concentration of surfactant according to the present invention is preferably 10% (all by weight of the total active material). Typically, the active material will constitute two actives e.g. levosalbutamol and the glucocorticoid.

In the compositions for inhalation particle size is particularly important. The preferred particle size is between 2 µm to 5µm. It has also been found that the particle size has a considerable influence on the proportion of active substance in the aerosol which is delivered for inhalation.

In another trial, drugs were mixed with propellant 11 or propellant 114 or a combination thereof, filled in canisters, crimped and charged with propellant 12. It was found that this gave a low FPD (fine particle dose). Hence further trials were undertaken where both the drugs and/or surfactant were micro-milled with propellant 11 or propellant 114 or a combination thereof to form a slurry and then filled in canisters, charging with propellant 12. This resulted in a better FPD as compared to the CFC aerosols where the micro-milling as described herein was not done. Hence micro-milling is preferably done in order to achieve a better FPD.

In a broad aspect, the invention provides a process for the manufacture of a pharmaceutical composition comprising a therapeutically effective isomer of salbutamol or a salt, solvate, ester, derivative or polymorph thereof and a glucocorticoid in a propellant, which process comprises mixing the said ingredients to form said composition.

In a further embodiment of the present invention there is provided a process for the manufacture of a pharmaceutical aerosol composition comprising a therapeutically effective isomer of salbutamol or a salt, solvate, ester, derivative or polymorph thereof and a glucocorticoid which process comprises (a) adding both the drugs, optionally with surfactant, with either propellant 11 or propellant 114 or a combination thereof to a canister (b) crimping the canister with a suitable valve and (c) charging propellant 12 through the valve. Preferably, in step (a) one or more of the actives are milled or micromilled with the propellant.

In yet another preferred aspect of the present invention, an aerosol composition may comprise a therapeutically effective isomer of salbutamol or a salt, solvate, ester, derivative or polymorph thereof with a glucocorticoid and either 1,1,1,2-tetrafluoroethane (HFA134a) or 1,1,1,2,3,3,3-heptafluoroethane (HFA227) or a combination thereof.

In a further aspect of the present invention there is provided a process for the manufacture of the above aerosol composition which process comprises (a) adding the therapeutically effective isomer of salbutamol and glucocorticoid to a canister (b) crimping the canister with a metered valve (c) charging the canister with either 1,1,1,2-tetrafluoroethane (HFA134a) or 1,1,1,2,3,3,3-heptafluoroethane (HFA227) or a combination thereof. Optionally, in step (a), there may also be added a cosolvent or bulking agent; a surfactant; or a cosolvent and surfactant.

In another preferred aspect of the present invention the aerosol composition may comprise a therapeutically effective isomer of salbutamol or a salt, solvate, ester, derivative or polymorph thereof with a glucocorticoid, either 1,1,1,2-tetrafluoroethane (HFA134a) or 1,1,1,2,3,3,3-heptafluoroethane (HFA227) or a combination thereof and a cosolvent. In such a case the cosolvent has a greater polarity than the propellant. Typically the cosolvent is present in an amount of 0.01 to 5 % by weight of the composition. The cosolvent used may be any suitable cosolvent ― for example selected from the group of glycols, particularly propylene glycol, polyethylene glycol and glycerol or alcohols like ethanol. Typically the cosolvent is ethanol.

In a preferred aspect of the present invention there is provided a process for the manufacture of the above composition which process comprises (a) adding both drugs to the canister (b) adding the cosolvent to (a) and sonicating (c) crimping the canister with a metered valve (d) charging the canister with either 1,1,1,2-tetrafluoroethane (HFA134a) or 1,1,1,2,3,3,3-heptafluoroethane (HFA227) or a combination thereof.

In yet another preferred embodiment, an aerosol composition may comprise a therapeutically effective isomer of salbutamol or a salt, solvate, ester, derivative or polymorph thereof with a glucocorticoid, and either 1,1,1,2-tetrafluoroethane (HFA134a) or 1,1,1,2,3,3,3-heptafluoroethane (HFA227) or a combination thereof, surfactant and cosolvent.

The surface-active agent (or surfactant) stabilizes the formulation and helps in the lubrication of a valve system in the inhaler. Some of the most commonly used surface active agents are those known in the art and be selected from among Polysorbate 20, Polysorbate 80, Myvacet 9-45, Myvacet 9-08, isopropylmyristate, oleic acid, Brij, ethyloleate, glyceryl trioleate, glyceryl monolaurate, glyceryl monooleate, glyceryl monosterate, glyceryl monoricinoleate, cetylalcohol, sterylalcohol, cetylpyridinium chloride, block polymers, natural oils, polyvinyl pyrrolidone, sorbitan fatty acid esters such as sorbitan trioleate, polyethoxylated sorbitan fatty acid esters (for example polyethoxylated sorbitan trioleate), sorbimacrogol oleate, synthetic amphotensides (tritons), ethylene oxide ethers of octylphenolformaldehyde condensation products, phosphatides such as lecithin, polyethoxylated fats, polyethoxylated oleotriglycerides and polyethoxylated fatty alcohols.

The surface-active agents are preferably used in an amount of 0.02-10% by weight of the total amount of active material.

In another aspect of the present invention there is provided a process for the manufacture of the above composition which process comprises (a) adding the drugs to a canister (b) adding cosolvent and surfactant to (a) and sonicating (c) crimping the canister with a metered valve (d) charging the canister with either 1,1,1,2-tetrafluoroethane (HFA134a) or 1,1,1,2,3,3,3-heptafluoroethane (HFA227) or a combination thereof.

In yet another aspect of the present invention the aerosol composition may comprise a therapeutically effective isomer of salbutamol, a glucocorticoid, a bulking agent and a propellant, which is preferably HFA 134a or HFA 227 or a combination thereof. The bulking agent acts as a carrier for the drug to reach the lungs. The bulking agent may be present in a concentration of 10-500% by weight of the total amount of active material. More preferably in a range of 10-300% by weight of the total amount of active material. The bulking agent may be selected from the class of saccharides, including monosaccharides, disaccharides, polysaccharides and sugar alcohols such as arabinose, glucose, fructose, ribose, mannose, sucrose, trehalose, lactose, maltose, starches, dextran or mannitol.

In a preferred aspect of the present invention there is provided a process for the manufacture of the above aerosol composition which process comprises (a) adding the active ingredients to a canister (b) adding a bulking agent to (a) (c) crimping the canister with a metered valve (d) charging the canister with propellant.

In a preferred aspect of the present invention the aerosol composition may comprise at least one therapeutically effective isomer of salbutamol or a salt, solvate, ester, derivative or polymorph thereof, a glucocorticoid, a surfactant and either 1,1,1,2-tetrafluoroethane (HFA134a) or 1,1,1,2,3,3,3-heptafluoroethane (HFA227) or a combination thereof. The surfactant may be any suitable surfactant ― for example those listed above or selected from the class of salts of stearic acids or esters such as ascorbyl palmitate, isopropyl myristate and tocopherol esters. Preferably the magnesium salt of stearic acid, isopropyl myristate. The surfactant is preferably used in an amount of 0.01 % to 1% by weight of the total amount of active material.

In a preferred aspect of the present invention there is provided a process for the manufacture of the above aerosol composition which process comprises (a) adding the drugs to a canister (b) adding surfactant to (a) (c) crimping the canister with a metered valve (d) charging the canister with either 1,1,1,2-tetrafluoroethane (HFA134a) or 1,1,1,2,3,3,3-heptafluoroethane (HFA227) or a combination thereof.

The compositions of the present invention may optionally contain antioxidants such as citric acid, or benzalkonium chloride.

The combination of levosalbutamol and a glucocorticoid may be provided as a dry powder formulation or in the form of an inhalation solution/suspension. For dry powder inhalation, the drugs may be used alone or optionally together with a finely divided pharmaceutically acceptable carrier, which is preferably present and may be chosen from materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran or mannitol. An especially preferred carrier is lactose. The dry powder may be in capsules of gelatin or HPMC, or in blisters or alternatively, the dry powder may be contained as a reservoir in a multi-dose dry powder inhalation device. The particle size of the active ingredient and that of the carrier where present in dry powder compositions, can be reduced to the desired level by conventional methods, for example by grinding in an air-jet mill, ball mill or vibrator mill, microprecipitation, spray-drying, lyophilisation or recrystallisation from supercritical media.

According to the present invention there is also provided a process for manufacture of a dry powder inhaler comprising levosalbutamol and a glucocorticoid, which process comprises mixing the active ingredients optionally with a suitable carrier, and providing the ingredients in a suitable dry powder inhaler.

For inhalation solutions, the drugs may be combined with suitable excipients such as tonicity adjusting agents, pH regulators, chelating agents, wetting agents in a suitable vehicle. The preferred tonicity adjusting agent is sodium chloride. The pH regulators may be selected from pharmacologically acceptable inorganic acids or organic acids or bases. Preferred inorganic acids are selected from the group consisting of hydrochloric acid, hydrobromic acid, nitric acid, sulphuric acid, phosphoric acid and the like. Preferred organic acids and salts of organic acids, such as but not limited to acetates, lactates, malates, tartrates, citrates, ascorbates, succinates, butyrates, valerates and fumarates. Preferred inorganic bases are selected from the group consisting of sodium hydroxide, potassium hydroxide, ammonium hydroxide, sodium carbonate, calcium hydroxide. Preferred organic bases are selected from the group consisting of methyl amine, ethyleneimine, hydroquinone, ethyleneimine, ethylamine, dimethylamine, ethanolamine, butylamine, diethylamine. The preferred base is sodium hydroxide. Preferably a nasal inhalation formulation as provided by the present invention has a pH in the range of 3 to 5.

Suitable chelating or complexing agents may be used in the compositions of the present invention, and may be molecules which are capable of entering into complex bonds. Preferable those compounds should have the effect of complexing cations most preferably metal cations, The preferred agent is ethylenediaminetetraacetic acid (EDTA) or a salt thereof, such as the disodium salt. Suitable wetting agents may be used in the present invention with good emulsifying and wetting properties. Some typical examples include sorbitan esters, PEG, etc which are obvious to a person skilled in the art.

Liquid vehicles for use in the compositions of the invention (particularly inhalation solutions or suspensions) include, but are not limited to, polar solvents, including, but not limited to, compounds that contain hydroxyl groups or other polar groups. Such solvents include, but are not limited to, water or alcohols, such as ethanol, isopropanol, and glycols including propylene glycol, polyethylene glycol, polypropylene glycol, glycol ether, glycerol and polyoxyethylene alcohols.

Further polar solvents also include protic solvents, including, but not limited to, water, aqueous saline solutions with one or more pharmaceutically acceptable salt(s), alcohols, glycols or a mixture thereof. For a saline solution as the solvent or as a component thereof, particularly suitable salts are those which display no or only negligible pharmacological activity after administration.

An Anti-microbial preservative agent may be added for multi-dose packages. Suitable preservatives will be apparent to the skilled person, particularly benzalkonium chloride or benzoic acid or benzoates such as sodium benzoate, sorbic acid or sorbates such as potassium sorbates in the concentration known from the prior art.

According to the present invention there is also provided a process for the manufacture of an inhalation solution comprising levosalbutamol and glucocorticoid. The process preferably comprises the following steps :
1. Dissolving levosalbutamol along with isotonocity agent, chelating agent and wetting agent in purified water followed by filtration.
2. In another vessel, sonication of the glucocorticoid in part quantity of water followed by appropriate sterilization method.
3. Both the above solutions are mixed to provide the final inhalation suspension and the pH is adjusted (if required). The suspension is filled in unit dose or multidose vials.

In another alternative embodiment, the inhalation solution of the present invention may be administered by nebulizer. Such nebulizer including, but not limited to, a jet nebulizer, ultrasonic nebulizer and breath actuated nebulizer. Preferably, the nebulizer is a jet nebulizer connected to an air compressor with adequate air flow. The nebulizer being equipped with a mouthpiece or suitable face mask. Specifically, a nebulizer (with face mask or mouthpiece) connected to a compressor may be used to deliver the inhalation solution of the present invention to a patient.

The present invention further provides for a method for the treatment in a mammal, such as a human, of respiratory disorders such as asthma, and disorders resulting in bronchoconstriction, which method comprises administration of a therapeutically effective amount of a pharmaceutical composition according to present invention.

It will be readily apparent to one skilled in the art that varying substitutions and modifications may be made to the invention disclosed herein without departing from the spirit of the invention. Thus, it should be understood that although the present invention has been specifically disclosed by the preferred embodiments and optional features, modification and variation of the concepts herein disclosed may be resorted to by those skilled in the art, and such modifications and variations are considered to be falling within the scope of the invention.

The following examples are for the purpose of illustration of the invention only and are not intended in any way to limit the scope of the present invention.

### Example 1: CFC inhaler A)

| Sr.No | Ingredients | Qty /can |
|---|---|---|
| 1. | Levo-Salbutamol Sulphate | 10.08 mg |
| 2. | Fluticasone Propionate | 8.24 mg |
| | (micro-milled) | |
| 3. | Lecithin 10% | 1.832 mg |
| 4. | Propellant 11 | 3.0 gms |
| 5. | Propellant 12 | 7.7 gms |

a) Add Levosalbutamol sulphate and lecithin with propellant 11
(b) Fill the slurry in the canisters.
(c) Crimp with a suitable valve and
(d) Charge propellant 12 through the valve.

### B)

| Sr.No | Ingredients | Qty /can |
|---|---|---|
| 1. | Levo-Salbutamol Sulphate | 15.12 mg |
| 2. | Beclomethasone Propionate (50 mcg) | 12 mg |
| 3. | Oleic acid 10% | 2.712 mg |
| 4. | Propellant 11 | 4.7 gms |
| 5. | Propellant 12 | 11.6 gms |

a) Add the drugs and oleic acid with propellant 11
b) (b) Fill the slurry in the canisters.
c) (c) Crimp with a suitable valve and
d) (d) Charge propellant 12 through the valve.

### C)

| Sr.No | Ingredients | Qty /can |
|---|---|---|
| 1. | Levo-Salbutamol Sulphate | 10.08mg |
| 2. | Budesonide | 24 mg |
| 3. | Lecithin 10% | 3.40 mg |
| 4. | Propellant 11 | 4.7 gms |
| 5. | Propellant 12 | 11.6 gms |

a) Add the drugs and lecithin with propellant 11
b) (b) Fill the slurry in the canisters.
c) (c) Crimp with a suitable valve and
d) (d) Charge propellant 12 through the valve.

### Example 2: HFA inhaler

### A)

| Sr.No | Ingredients | Qty /can |
|---|---|---|
| 1. | Levosalbutamol sulphate | 12.00mg |
| 2. | Fluticasone Propionate (micro-milled)(50 mcg) | 8.24 mg |
| 3. | Propellant 134a | 12.8gm |

a) Add both the drugs to the canister.
b) Crimp the canister with a metered valve
c) Charge the canister with 1,1,1,2-tetrafluoroethane (HFA 134a) .

### B)

| Sr.No | Ingredients | Qty /can |
|---|---|---|
| 1. | Levo-Salbutamol Sulphate | 15.12 mg |
| 2. | Beclomethasone Propionate(50 mcg) | 12 mg |
| 3. | Abs. Alc.2.5% | 0.455 |
| 4. | HFA 134a | 17.74 gms |

a) Add both the drugs and alcohol and a part of HFA134a to the canister.
b) Crimp the canister with a metered valve and sonicate.
c) Charge the canister with 1,1,1,2-tetrafluoroethane (HFA134a) .

### C)

| Sr.No | Ingredients | Qty /can |
|---|---|---|
| 1. | Levo-Salbutamol Sulphate | 10.08 mg |
| 2. | Budesonide(100 mcg) | 24 mg |
| 3. | HFA 134a | 18.2 gms |

a) Add both the drugs to the canister.
b) Crimp the canister with a metered valve
c) Charge the canister with 1,1,1,2-tetrafluoroethane (HFA134a) .

### Example 3: HFA inhaler

### A)

| Sr.No | Ingredients | Qty /can |
|---|---|---|
| 1. | Levosalbutamol sulphate | 10.08mg |
| 2. | Fluticasone Propionate (micro-milled) | 8.24 mg |
| 3. | Propellant 227 | 11.2 gms |

a) Add both the drugs to the canister.
b) Crimp the canister with a metered valve
c) Charge the canister with 1,1,1,2,3,3,3-heptafluoroethane (HFA227)

### B)

| Sr.No | Ingredients | Qty /can |
|---|---|---|
| 1. | Levosalbutamol sulphate | 10.08mg |
| 2. | Budesonide | 24 mg |
| 3. | HFA 227 | 20.6 gms |

a) Add both the drugs to the canister.
b) Crimp the canister with a metered valve
c) Charge the canister with 1,1,1,2,3,3,3-heptafluoroethane (HFA227)

### Example 4: HFA inhaler

### A)

| Sr.No | Ingredients | Qty /can |
|---|---|---|
| 1. | Levo-Salbutamol Sulphate | 10.08 mg |
| 2. | Fluticasone Propionate (micro-milled) | 8.24 mg |
| 3. | Abs. Alc.2% | 0.256 |
| 4. | Lecithin 0.02% | 0.003664 mg |
| 5. | HFA 134a | 12.54 gms |

a) Add both the drugs to the canister.
b) Add alcohol and surfactant solution to (a) and sonicate
c) Crimp the canister with a metered valve
d) Charge the canister with 1,1,1,2-tetrafluoroethane (HFA134a).

### B)

| Sr.No | Ingredients | Qty /can |
|---|---|---|
| 1. | Levo-Salbutamol Sulphate | 15.12 mg |
| 2. | Beclomethasone Propionate(50 mcg) | 12 mg |
| 3. | Abs. Alc.2.5% | 0.455 |
| 4. | Oleic acid 0.02% | 0.00542 |
| 5. | HFA 134a | 17.74 gms |

a) Add both the drugs to the canister.
b) Add alcohol and surfactant solution to (a) and sonicate
c) Crimp the canister with a metered valve
d) Charge the canister with 1,1,1,2-tetrafluoroethane (HFA134a).

### C)

| Sr.No | Ingredients | Qty /can |
|---|---|---|
| 1. | Levo-Salbutamol Sulphate | 10.08 mg |
| 2. | Budesonide | 24 mg |
| 3. | Abs. Alc.2% | 0.364 |
| 4. | Lecithin 0.02% | 0.006816 mg |
| 5. | HFA 134a | 17.83 gms |

a) Add both the drugs to the canister.
b) Add alcohol and surfactant solution to (a) and sonicate of the same
c) Crimp the canister with a metered valve
d) Charge the canister with 1,1,1,2-tetrafluoroethane (HFA134a).

### Example 5: Dry powder for inhalation

| Sr.No | Ingredients | mg/cap |
|---|---|---|
| 1. | Levo-Salbutamol Sulphate | 100.00 mcg |
| 2. | Beclomethasone dipropionate | 100.00 mcg |
| 3. | Lactose q.s. | 25.00 mg |

Levosalbutamol sulphate and beclomethasone dipropionate are blended together with lactose and filled in capsules

### Example 6: Nebulising suspension

| Sr.No | Ingredients | Quantity (%w/w) |
|---|---|---|
| 1. | Levo-Salbutamol Sulphate equiv to levosalbutamol | 15.500 |
| 2. | Beclomethasone dipropionate | 20.000 |
| 3. | Sodium chloride | 0.900 |
| 4. | Tween 80 | 0.100 |
| 5. | Disodium edetate | 0.020 |
| 6. | Sodium citrate | q.s |
| 7. | Purified water | q.s. to 2.00ml |

| | | |
|---|---|---|
| 1. Dissolving levosalbutamol along with isotonocity agent, chelating agent and wetting agent in purified water followed by filtration. 2. In another vessel, sonication of the glucocorticoid in part quantity of water followed by appropriate sterilization method. 3. Both the above solutions are mixed to provide the final inhalation suspension and the pH is adjusted (if required). The suspension is filled in unit dose or multidose vials. | | |

The following Examples illustrate preparation of crystalline polymorphic Forms I, II and III of levosalbutamol sulphate.

### Example 7

R-benzyl salbutamol (20.0 kg.), methanol ( 61.0 Itr.), denatured alcohol (72 Itrs.) was charged in an autoclave, palladium (5%) on charcoal (1.30 kg) was charged and stirred under 30 psi hydrogen pressure. After completion of reaction the catalyst was filtered and washed with methanol (60 Its.) and denatured alcohol (60 Itrs.). The pH of the clear filtrate was adjusted with sulphuric acid to 5 -5.5 pH at 0-10°C and the resulting solid was stirred at 0-10°C for 1 hr., filtered and washed with methanol (20 ltrs.). The product was dried under vacuum at 30°C for 1 hr. and further at 50-60°C for additional 1 hr. to give R-salbutamol Form I ( 19.0 kg.).

### Example 8

R-benzyl salbutamol (10.0 kg.), methanol (30.0 Itr.), denatured alcohol (36 ltrs.) was charged in an autoclave, wet palladium (5%) on charcoal (0.65 kg) was charged and stirred under 30 psi hydrogen pressure. After completion of reaction the catalyst was filtered and washed with denatured alcohol (25 ltrs.). The pH of the clear filtrate was adjusted with sulphuric acid to 5 -5.5 pH at ambient temperature (25 to 30° C) and the resulting solid was filtered and washed with methanol (10 ltrs.) at 25 to 30° C. The product was dried under vacuum at 50-60°C temp to give R-salbutamol sulphate Form III ( 19.0 kg.).

### Example 9

R-salbutamol sulphate (14.80 Kg) was dissolved in water (60.0 ltrs.) and filtered to get a clear solution. The filtrate was distilled under vacuum below 60°C to residue. The residue was stripped with acetone (74.0 ltrs.) twice, further acetone (148.0 lts.) was added and the resulting slurry was stirred for 2 hrs. The slurry was filtered and dried under vacuum at 60°C for 10-12 hrs to give R-salbutamol sulphate Form II (11.1 kg.)

### Example 10

R-salbutamol sulphate (10 Kg) was dissolved in water (30.0 ltrs.) and stirred for 10-15 min. The resulting clear solution was filtered. Methanol (150 ltrs.) was added slowly to the clear filtrate at room temperature and stirred for 30 mins. and further chilled to 0-5°C. The resulting solid was filtered and washed with methanol. The product was dried under vacuum at 60°C for 3-4 hrs to give R-salbutamol sulphate Form I (8 kg.)

### Example 11

R-salbutamol sulphate (20 Kg) was dissolved in water (60.0 ltrs.) and filtered to get a clear solution ,charge 300 ltr acetone slowly at 25-30°C and the resulting mixture was stirred for 2 hrs at room temp. The resulting slurry was filtered and dried under vacuum at 80°C for 10-12 hrs to give R-salbutamol sulphate Form III (17 kg.)

### Example 12

R-salbutamol sulphate (10 gms) was dissolved in water (30 ml). Methanol (150 ml) was charged at 25-30°C and Isopropyl alcohol (75ml) was added and the mixture was cooled to 5-10°C for 2 hrs. filtered and dried at 80°C under vacuum for 15-20 hrs. to give Form II.

### Example 13

R-salbutamol sulphate was dissolved in methanol at reflux temperature. The reaction mass was then cooled to room temperature and further chilled to 5-10°C . The resulting solid was filtered and dried at 80°C to give R-salbutamol sulphate Form II

### Example 14

R-salbutamol sulphate Form I was subjected to jet milling to get R-salbutamol sulphate Form II having a particle size of 90% less than 5 micron and 100 % below 12.5 micron.

Note that in Examples 9 to 13 any form of R-salbutamol sulphate may be used as the stating material.

**TABLE 1**

| **LEVOSALBUTAMOL S04 - Form I** | | | | | |
|---|---|---|---|---|---|
| | | | | | |

| Peak No. | 2θ (deg) | d (A) | Height | Height % | FWHM |
|---|---|---|---|---|---|
| 1 | 10.781 | 8.1998 | 10389 | 59.5 | 0.237 |
| 2 | 11.941 | 7.4053 | 2043 | 11.7 | 0.237 |
| 3 | 12.660 | 6.9865 | 1090 | 6.2 | 0.232 |
| 4 | 13.005 | 6.8036 | 1080 | 6.2 | 0.167 |
| 5 | 15.819 | 5.5975 | 1576 | 9.0 | 0.266 |
| 6 | 17.400 | 5.0924 | 2170 | 12.4 | 0.236 |
| 7 | 18.341 | 4.8332 | 2847 | 16.3 | 0.268 |
| 8 | 19.019 | 4.6624 | 621 | 3.6 | 0.271 |
| 9 | 20.939 | 4.2390 | 2564 | 14.7 | 0.265 |
| 10 | 21.720 | 4.0883 | 3195 | 18.3 | 0.282 |
| 11 | 22.500 | 3.9482 | 2001 | 11.5 | 0.202 |
| 12 | 23.140 | 3.8406 | 17446 | 100.0 | 0.234 |
| 13 | 24.341 | 3.6537 | 1870 | 10.7 | 0.243 |
| 14 | 26.120 | 3.4087 | 1108 | 6.4 | 0.285 |
| 15 | 28.541 | 3.1249 | 1379 | 7.9 | 0.281 |
| 16 | 31.280 | 2.8572 | 914 | 5.2 | 0.378 |
| 17 | 31.939 | 2.7997 | 955 | 5.5 | 0.451 |
| 18 | 33.980 | 2.6361 | 686 | 3.9 | 0.361 |
| 19 | 34.279 | 2.6138 | 419 | 2.4 | 0.350 |
| 20 | 35.739 | 2.51036 | 712 | 4.1 | 0.329 |
| 21 | 36.340 | 2.4702 | 635 | 3.6 | 0.391 |

**TABLE 2**

| **LEVOSALBUTAMOL S04 - Form II** | | | | | |
|---|---|---|---|---|---|
| | | | | | |

| Peak No. | 2θ (deg) | d (A) | Height | Height % | FWHM |
|---|---|---|---|---|---|
| 1 | 8.701 | 10.1542 | 8249 | 100.0 | 0.205 |
| 2 | 9.636 | 9.1706 | 2610 | 31.6 | 0.195 |
| 3 | 13.422 | 6.5914 | 365 | 4.4 | 0.184 |
| 4 | 15.180 | 5.8318 | 6090 | 73.8 | 0.213 |
| 5 | 15.657 | 5.6550 | 2247 | 27.2 | 0.201 |
| 6 | 17.440 | 5.0809 | 2091 | 25.3 | 0.193 |
| 7 | 19.139 | 4.6335 | 1416 | 17.2 | 0.272 |
| 8 | 19.360 | 4.5811 | 900 | 10.9 | 0.385 |
| 9 | 19.583 | 4.5294 | 666 | 8.1 | 0.376 |
| 10 | 20.221 | 4.3879 | 462 | 5.6 | 0.156 |
| 11 | 21.439 | 4.1413 | 7819 | 94.8 | 0.256 |
| 12 | 21.699 | 4.0921 | 3525 | 42.7 | 0.356 |
| 13 | 22.201 | 4.0008 | 2317 | 28.1 | 0.128 |
| 14 | 22.837 | 3.8907 | 1299 | 15.7 | 0.091 |
| 15 | 23.339 | 3.8083 | 4096 | 49.7 | 0.308 |
| 16 | 23.760 | 3.7417 | 2345 | 28.4 | 0.236 |
| 17 | 24.361 | 3.6508 | 1107 | 13.4 | 0.165 |
| 18 | 25.022 | 3.5558 | 829 | 10.0 | 0.080 |
| 19 | 25.399 | 3.5038 | 1127 | 13.7 | 0.176 |
| 20 | 26.059 | 3.4166 | 1162 | 14.1 | 0.271 |
| 21 | 26.321 | 3.3832 | 1437 | 17.4 | 0.256 |
| 22 | 27.199 | 3.2759 | 2718 | 32.9 | 0.255 |
| 23 | 28.740 | 3.1037 | 622 | 7.5 | 0.193 |
| 24 | 29.263 | 3.0493 | 356 | 4.3 | 0.628 |
| 25 | 30.077 | 2.9687 | 721 | 8.7 | 0.162 |
| 26 | 30.702 | 2.9097 | 1586 | 19.2 | 0.211 |
| 27 | 31.640 | 2.8255 | 631 | 7.6 | 0.351 |
| 28 | 32.001 | 2.7944 | 700 | 8.5 | 0.464 |
| 29 | 32.319 | 2.7677 | 680 | 8.2 | 0.354 |
| 30 | 33.859 | 2.6452 | 368 | 4.5 | 0.382 |
| 31 | 34.242 | 2.6165 | 730 | 8.8 | 0.315 |
| 32 | 35.002 | 2.5615 | 424 | 5.1 | 0.244 |
| 33 | 35.299 | 2.5406 | 316 | 3.8 | 0.542 |
| 34 | 35.838 | 2.5036 | 376 | 4.6 | 0.239 |
| 35 | 36.238 | 2.4769 | 427 | 5.2 | 0.232 |
| 36 | 36.737 | 2.443 | 254 | 3.1 | 0.313 |
| 37 | 37.999 | 2.3660 | 297 | 3.6 | 0.245 |
| 38 | 38.265 | 2.3502 | 319 | 3.9 | 0.658 |
| 39 | 38.777 | 2.3203 | 491 | 6.0 | 0.380 |

**TABLE 3**

| **LEVOSALBUTAMOL S04 - Form III** | | | | | |
|---|---|---|---|---|---|
| | | | | | |

| Peak No. | 2θ (deg) | d (A) | Height | Height % | FWHM |
|---|---|---|---|---|---|
| 1 | 5.496 | 16.0657 | 2337 | 41.8 | 0.206 |
| 2 | 6.901 | 12.799 | 320 | 5.7 | 0.295 |
| 3 | 7.340 | 12.034 | 1938 | 34.6 | 0.217 |
| 4 | 8.181 | 10.7983 | 2348 | 42.0 | 0.645 |
| 5 | 8.399 | 10.5187 | 5559 | 99.4 | 0.251 |
| 6 | 10.978 | 8.0527 | 577 | 10.3 | 0.190 |
| 7 | 11.758 | 7.5203 | 978 | 17.5 | 0.178 |
| 8 | 12.778 | 6.9221 | 365 | 6.5 | 0.186 |
| 9 | 14.298 | 6.1895 | 565 | 10.1 | 0.233 |
| 10 | 14.701 | 6.0206 | 428 | 7.7 | 0.165 |
| 11 | 16.321 | 5.4266 | 4839 | 86.5 | 0.292 |
| 12 | 16.981 | 5.2172 | 498 | 8.9 | 0.134 |
| 13 | 17.980 | 4.9293 | 1110 | 19.8 | 0.319 |
| 14 | 18.180 | 4.8758 | 1421 | 25.4 | 0.532 |
| 15 | 18.660 | 4.7512 | 4455 | 79.6 | 0.432 |
| 16 | 18.860 | 4.7013 | 3247 | 58.0 | 0.243 |
| 17 | 19.189 | 4.6215 | 636 | 11.4 | 0.100 |
| 18 | 20.179 | 4.3969 | 797 | 14.2 | 0.529 |
| 19 | 20.720 | 4.2833 | 2355 | 42.1 | 0.315 |
| 20 | 22.019 | 4.0335 | 5594 | 100.0 | 0.306 |
| 21 | 22.219 | 3.9976 | 2598 | 46.4 | 0.595 |
| 22 | 23.121 | 3.8436 | 761 | 13.6 | 0.563 |
| 23 | 23.640 | 3.7604 | 2729 | 48.8 | 0.460 |
| 24 | 23.858 | 3.7265 | 2189 | 39.1 | 0.547 |
| 25 | 24.638 | 3.6103 | 654 | 11.7 | 0.168 |
| 26 | 25.339 | 3.5120 | 1235 | 22.1 | 0.276 |
| 27 | 25.721 | 3.4607 | 445 | 8.0 | 0.215 |
| 28 | 26.299 | 3.3859 | 414 | 7.4 | 0.352 |
| 29 | 26.518 | 3.3585 | 550 | 9.8 | 0.354 |
| 30 | 26.879 | 3.3142 | 493 | 8.8 | 0.249 |
| 31 | 27.620 | 3.2270 | 1316 | 23.5 | 0.274 |
| 32 | 28.799 | 3.0974 | 719 | 12.9 | 0.655 |
| 33 | 29.319 | 3.0437 | 827 | 14.8 | 0.654 |
| 34 | 30.800 | 2.9006 | 565 | 10.1 | 0.319 |
| 35 | 31.242 | 2.8606 | 430 | 7.7 | 0.207 |
| 36 | 32.341 | 2.7659 | 867 | 15.5 | 0.232 |
| 37 | 33.218 | 2.6948 | 719 | 12.9 | 0.313 |
| 38 | 33.781 | 2.6512 | 565 | 10.1 | 0.245 |
| 39 | 34.181 | 2.6211 | 1029 | 18.4 | 0.267 |
| 40 | 36.646 | 2.4502 | 325 | 5.8 | 0.557 |
| 41 | 37.140 | 2.4187 | 376 | 6.7 | 0.252 |
| 42 | 37.522 | 2.3950 | 478 | 8.5 | 0.306 |
| 43 | 39.397 | 2.2852 | 356 | 6.4 | 0.427 |

## Claims

1. Crystalline levosalbutamol sulphate (Form II) **characterised by** a powder XRD pattern with peaks at 8.7, 9.6, 15.2, 15.7, 19.1, 27.2, 30.7 ± 0.2 degrees 2 theta.

2. Crystalline levosalbutamol sulphate according to claim 1 **characterised by** an IR spectrum with peaks at 3393, 3026, 2982, 2822, 2463, 1630, 1614, 1513, 1484, 1448, 1380, 1321, 1279, 1258, 1235, 1204, 1155, 1093, 1066, 1036, 1023, 919, 900, 838, 829, 818, 808, 788, 618, 596, 540, 493, 453, 440 cm⁻¹.

3. Crystalline levosalbutamol sulphate according to claim 1 or 2 substantially as shown in Figure 3; and/or
**characterised by** having an IR spectrum substantially as shown in Figure 4; and/or
**characterised by** a powder XRD pattern with peaks substantially as shown in Table 2.

4. A process for preparing crystalline levosalbutamol sulphate Form II according to claim 1 or 2 which process comprises a) dissolving any form of levosalbutamol sulphate in water b) distilling to residue c) stripping the residue with an organic solvent d) slurrying the solid in an organic solvent e) isolating crystalline Form II.

5. A process according to claim 4 wherein step (a) comprises dissolving crystalline levosalbutamol sulphate Form I or Form II in water.

6. A process according to claim 4 or 5 wherein in step (c) or step (d) or both the solvent is acetone.

7. A process for preparing crystalline levosalbutamol sulphate Form II according to claim 1, 2 or 3, which process comprises jet milling any other form of levosalbutamol sulphate.

8. A process according to claim 7 wherein crystalline levosalbutamol Form I is jet milled to give said Form II.

9. A pharmaceutical composition comprising a compound according to any one of claims 1 to 3, and a pharmaceutically acceptable carrier.

10. A pharmaceutical composition comprising a compound according to any one of claims 1 to 3 or a compound prepared by the process of any one of claims 4 to 8 in combination with one or more pharmaceutically active compounds and, optionally, a pharmaceutically acceptable carrier.

11. A pharmaceutical composition according to claim 10 wherein the further active compound is a glucocorticoid, optionally wherein the glucocorticoid is one or more of fluticasone propionate, beclomethasone dipropionate or budesonide.

12. A compound according to any one of claims 1 to 3, or a composition according to claim 9, 10 or 11, for use as a medicament.

13. A compound or composition according to claim 12 for use in the treatment of respiratory disorders and related conditions.

14. A combination comprising a compound according to any one of claims 1 to 3 and one or more pharmaceutically active compounds and optionally at least one pharmaceutically acceptable carrier; for simultaneous, separate or sequential use.

15. A pharmaceutical composition according to any one of claims 9 to 14 comprising suitable pharmaceutically acceptable excipients to form an aerosol formulation, a dry powder formulation or an inhalation solution/suspension.

16. A pharmaceutical composition according to claim 15 further comprising a propellant selected from the group comprising propellant 11, propellant 12, propellant 114, 1,1,1,2-tetrafluoroethane (HFA134a) and 1,1,1,2,3,3,3-heptafluoropropane (HFA227), or mixtures of two or more such halogen-substituted hydrocarbons.; optionally a surfactant is optionally a cosolvant; and optionally a bulking agent.

17. A pharmaceutical composition according to claim 15 or 16 in the form of a dry powder formulation.

18. A dry powder inhaler comprising a composition according to claim 17.

19. A pharmaceutical composition according to claim 15 in the form of an inhalation suspension.

20. A pharmaceutical composition according to claim 19 comprising, in addition to active material, a polar solvent, a tonicity-adjusting agent, a wetting agent, a chelating agent and optionally an acid.

21. A composition according to any one of claims 15 to 20 for use as a medicament.

22. A composition according to any one of claims 15 to 20 for use in treating respiratory disorders and related conditions, including bronchoconstriction, asthma and COPD.

23. Use of a combination of crystalline levosalbutamol sulphate Form II according to any one of claims 1 to 3 and a glucocorticoid for treatment in the long-term management of asthma and COPD.

24. A combination comprising levosalbutamol sulphate Form II according to any one of claims 1 to 3 and a glucocorticoid and optionally at least one pharmaceutically acceptable carrier; for simultaneous, separate or sequential use.
